Europäisches Patentamt

European Patent Office

Office européen des brevets

(11) Veröffentlichungsnummer : **0 061 688**
**B1**

(12) # EUROPÄISCHE PATENTSCHRIFT

(45) Veröffentlichungstag der Patentschrift :
28.08.85

(21) Anmeldenummer : 82102356.1

(22) Anmeldetag : 22.03.82

(51) Int. Cl.⁴ : **C 12 P 33/00// C07J9/00**

(54) Verfahren zum gezielten mikrobiologischen Abbau von Gallensäuren.

(30) Priorität : 01.04.81 DE 3112981

(43) Veröffentlichungstag der Anmeldung :
06.10.82 Patentblatt 82/40

(45) Bekanntmachung des Hinweises auf die Patenterteilung : 28.08.85 Patentblatt 85/35

(84) Benannte Vertragsstaaten :
AT CH DE FR GB LI NL

(56) Entgegenhaltungen :
EP-A- 0 004 913
EP-A- 0 015 308
WO-A-81 /024 27
CHEMICAL ABSTRACTS, Band 89, Nr. 19, 6. November 1978, Seite 275, Nr. 159886C, Columbus, Ohio,
USA M.E. TENNESON et al.: "The degradation of
lithocholic acid by Pseudomonas Spp NCIB 10590"

(73) Patentinhaber : Henkel Kommanditgesellschaft auf
Aktien
Postfach 1100 Henkelstrasse 67
D-4000 Düsseldorf-Holthausen (DE)

(72) Erfinder : Bahn, Michael, Dr.
Frans-Hals-Weg 19
D-4010 Hilden (DE)
Erfinder : Schindler, Joachim, Dr.
Am Eichelkamp 158
D-4010 Hilden (DE)
Erfinder : Schmid, Rolf, Dr.
Am Nettchesfeld 30
D-4000 Düsseldorf 13 (DE)

**0 061 688**

**Beschreibung**

Die technische Herstellung pharmakologisch aktiver Steroidverbindungen, beispielsweise solcher der Progesteron- oder Dehydroprogesteron-Serie benutzt natürlich vorkommende Grundstoffe mit Steroidstruktur als Ausgangsmaterial der Synthese. Durch chemische Konversion des üblicherweise in C-17 vorliegenden Substituenten und gegebenenfalls durch Einflußnahme auf die Ringstruktur können die erforderlichen Abänderungen des Moleküls vorgenommen werden, ohne daß die komplizierte und stereospezifische Mehrringstruktur als solche gefährdet wird.

In jüngster Zeit hat sich die Möglichkeit ergeben, für die großtechnische Synthese natürlich vorkommende Sterine pflanzlichen oder tierischen Ursprunges wie Cholesterin, Sitosterin, Stigmasterin und/oder Campesterin einzusetzen. Nach diesen Vorschlägen gelingt es, unter Erhalt der Steroid-Ringstruktur den üblicherweise 8 bis 10 C-Atome enthaltenden Substituenten in C-17 selektiv zum α-Propionsäurerest abzubauen. Gleichzeitig wird der A-Ring des Steroid-Moleküls zur 3-Oxo-4-en- bzw. zur 3-Oxo-1,4-dien-Struktur umgewandelt. Diese Möglichkeit ergibt sich durch ein mikrobiologisches Verfahren, das beispielsweise in den europäischen Patentanmeldungen 004913 und 0015308 geschildert ist. Durch Verwendung bestimmt ausgewählter Mikroorganismen-Defektmutanten gelingt es, beispielsweise aus Cholesterin oder Sitosterin 3-Oxo-pregna-4-en-20-carbonsäure und/oder 3-Oxo-pregna-1,4-dien-20-carbonsäure in hohen Ausbeuten herzustellen. Die dabei eingesetzten Mikroorganismen-Defektmutanten werden durch ein ganz bestimmtes Selektions-Mutations-Selektionsverfahren gewonnen. Hierzu werden zunächst bestimmt ausgewählte Mikroorganismenwildstämme isoliert und gezüchtet, die insbesondere durch eine zahlenmäßig definierte selektive Abbauleistung an C-17-Alkylsterinverbindungen gekennzeichnet sind. Die so ausgewählten Wildstämme werden dann einer bekannten Mutation unterworfen, woraufhin die Mutantenpopulation auf einem Trennmedium gezüchtet wird, auf dem die 17-C-Steroid-α-propionsäureverbindungen liefernden Mutanten praktisch nicht wachsen, während unerwünschte begleitende Mutantenstämme wachsen und hierdurch bzw. während ihres Wachstums abgetötet werden, woraufhin der verbleibende Rest der Mutantenstämme auf Sterinen wie Cholesterin oder Sitosterin gezüchtet und dabei die Stämme mit optimaler Produktion der BNC-Verbindungen isoliert werden. Bei Züchtung der solcherart ausgewählten Defektmutanten in einem wäßrigen Nährmedium unter aeroben Bedingungen un unter Verwendung von C-17-Alkylsterinen wie Cholesterin, Sitosterin und dergleichen als Kohlenstoffquelle für das Mikroorganismenwachstum fallen als gesuchtes Verfahrensprodukt die 3-Oxo-pregna-4-en-20-carbonsäure und/oder 3-Oxo-pregna-1,4-dien-20-carbonsäure in hohen Ausbeuten an.

Ein in der Natur gleichfalls in beträchtlicher Menge zur Verfügung stehendes Ausgangsmaterial mit der Steroid-Grundstruktur sind die Gallensäuren. Man erhält bekanntlich beispielsweise durch alkalische Hydrolyse von Galle ein Gemisch hochmolekularer Säuren, das sich aus den sogenannten Gallensäuren zusammensetzt. Den hauptsächlichen Anteil in tierischer Galle bildet die Cholsäure. In untergeordneten Mengen liegen als strukturverwandte Verbindungen Desoxycholsäure, Lithocholsäure und gegebenenfalls Chenodesoxycholsäure in Spuren vor.

Zwar leiten sich die Gallensäuren — ebenso wie die zuvor erwähnten C-17-Alkylsterinverbindungen (Cholesterin und dgl.) von der Steroidringstruktur ab, gleichwohl weisen die beiden hier betroffenen Stoffklassen beträchtliche prinzipielle Unterschiede auf und zwar insbesondere gerade an den Molekülstellen, die im eingangs geschilderten mikrobiologischen Abbauverfahren verändert werden sollen. Übereinstimmend besitzen die Gallensäuren in C-17 keinen Alkylrest wie Cholesterin, Sitosterin oder verwandte Verbindungen, sondern einen Carboxyalkylrest mit endständiger Carboxylgruppe in C-24. Während in Cholesterin und verwandten Sterinen der B-Ring in 5/6-Stellung ungesättigt ist, sind in den Gallensäuren der A- und der B-Ring gesättigt. Dabei sind die Ringe A und B in den Gallensäuren cisständig miteinander verknüpft. Cholesterin, Sitosterin und die anderen verwandten Alkylsterinverbindungen stellen 3-β-Oxysteroide dar, demgegenüber besitzen die Gallensäuren durchweg eine 3-α-Hydroxylgruppe. Cholsäure, Desoxycholsäure und Chenodesoxycholsäure besitzen neben der 3-α-Hydroxylgruppe weitere Hydroxylgruppensubstituenten im Ringgerüst. Die Cholsäure besitzt zwei weitere Hydroxylgruppen in 7- und 12-Stellung, die Desoxycholsäure besitzt eine weitere Hydroxylgruppe in 12-Stellung, die Chenodesoxycholsäure eine weitere Hydroxylgruppe in 7-Stellung.

Zur mikrobiologischen Transformation der Gallensäuren existiert ein umfangreicher Stand der Technik. Eine zusammenfassende Darstellung findet sich in « Advances in Lipid Research » Band 11 (1973) Seiten 143 - 192 (Academic Press, New York and London). Verwiesen wird insbesondere auf das Kapitel III (a. a. O. Seiten 162 ff.), in dem zahlreiche Reaktionsprodukte der mikrobiologischen Transformation formelmäßig dargestellt sind. Unter Angriff auf die Ringstruktur und/oder den Seitenkettensubstituenten in C-17 sind in Abhängigkeit vom jeweils eingesetzten Mikroorganismus die verschiedenartigsten Reaktionsprodukte mit Reststruktur-Bestandteilen der Gallensäuren erhalten worden. Gemäß Severina et al soll beim Arbeiten mit M. mucosum, Corynebacterium equi und Streptomyces gelaticus eine Mehrzahl von Reaktionsfolgeprodukten mit einem α-Propionsäurerest in C-17 und einer 3-Oxo-4-en-Struktur anfallen. Angaben für ein wiederholbares und großtechnisches brauchbares Verfahren zur gezielten Herstellung ganz bestimmter Abbauprodukte der Gallensäuren, die insbesondere wertvolle Zwischenprodukte für die weitere chemische Transformation beispielsweise zu Steroidverbindungen der Progesteron-Serie darstellen, finden sich hier jedoch nicht.

2

Die Erfindung geht von der Aufgabe aus, die gezielte selektive Umwandlung von Gallensäuren zu Transformationsprodukten zu ermöglichen, die den eingangs geschilderten Sterin-Transformationsprodukten 3-Oxo-pregna-4-en-20-carbonsäure und 3-Oxo-pregna-1,4-dien-20-carbonsäure strukturähnlich sind, dabei jedoch die für Gallensäuren charakteristischen zusätzlichen Sauerstoff-Funktionen im Ringgerüst aufweisen. Im Rahmen der technischen Lösung dieser erfindungsgemäßen Aufgabe hat sich überraschenderweise gezeigt, daß die für den selektiven C-17-Alkylsterinabbau entwickelten Mikroorganismen-Defektmutanten auch bei ihrem Einsatz auf Gallensäuren als Kohlenstoffquelle des Mikroorganismenwachstums hochaktiv und dabei weitgehend selektiv im angestrebten Sinne wirksam sind. Gerade unter Berücksichtigung der Unterschiede in der jeweiligen Molekülstruktur an den für den Enzymangriff entscheidenden Stellen zwischen Alkylsterinen einerseits und Gallensäuren andererseits sowie der üblicherweise gegebenen hochspezifischen Enzymwirkung mikrobiologischer Verfahren war das nicht vorherzusehen.

Gegenstand der Erfindung ist dementsprechend ein Verfahren zum mikrobiologischen Abbau von Gallensäuren durch aerobes Züchten von Mikroorganismen in einem wäßrigen Nährmedium unter Verwendung eines Gallensäuren enthaltenden Einsatzmaterials als Kohlenstoffquelle für das Mikroorganismen-Wachstum. Das neue Verfahren ist dadurch gekennzeichnet, daß man zum selektiven Abbau der Steroid-C-17-Carboxyalkylseitenkette zum α-Propionsäurerest und zur gleichzeitigen Umwandlung des Steroid-A-Ringes in die 3-Oxo-4-en- und/oder 3-Oxo-1,4-dien-Struktur mit Mikroorganismen-Defektmutanten arbeitet, die zum Wachstum auf pflanzlichen und/oder tierischen Sterinverbindungen mit einer C-17-Alkyl- bzw. -Alkenyl-Seitenkette als alleiniger Kohlenstoffquelle befähigt sind und aus diesen auch in Abwesenheit von den Steroidringabbau und/oder das Wachstum hemmenden Inhibitoren 3-Oxo-pregna-4-en-20-carbonsäure (Δ-4-BNC) und/oder 3-Oxo-pregna-1,4-dien-20-carbonsäure (Δ-1,4-BNC) bilden. Die Hydroxylgruppenfunktionen von Cholsäure und Desoxycholsäure in 12- und gegebenenfalls 7-Stellung bleiben entweder als solche erhalten oder werden ganz oder teilweise in entsprechende Oxofunktionen umgewandelt. Die als Verfahrensprodukt angefallenen BNC-Verbindungen können in konventioneller Weise aus dem Reaktionsgemisch isoliert werden.

Der Kern des erfindungsgemäßen Verfahrens liegt damit in der Anwendung der gemäß der Lehre der europäischen Patentanmeldungen 004913 bzw. 0015308 hergestellten bzw. zu gewinnenden Mikroorganismen-Defektmutanten auf ein Gallensäuren enthaltendes Ausgangsmaterial, mit dem Ziel, daraus 3-Oxo-pregna-4-en-20-carbonsäure und/oder 3-Oxo-pregna-1,4-dien-20-carbonsäure-Verbindungen zu bilden, die insbesondere in der Ringstruktur zusätzliche Sauerstoffunktionen aufweisen. Es war überraschend, daß die Anwendung dieser für den Abbau von Sterinverbindungen wie Cholesterin oder Sitosterin gewissermaßen maßgeschneiderten Mikroorganismen-Defektmutanten auf ein Einsatzmaterial wirkungsvoll möglich ist, das beträchtliche strukturelle Unterschiede gegenüber beispielsweise Cholesterin oder Sitosterin besitzt, und zwar insbesondere gerade an den Molekülstellen, an denen der mikrobiologische Angriff Ziel der beabsichtigten Reaktion ist.

Die technische Verwirklichung des erfindungsgemäßen Verfahrens gelingt in einfacher Weise derart, daß in das in den europäischen Patentanmeldungen 004913 und 0015308 geschilderte Verfahren zur Herstellung von BNC-Verbindungen an Stelle der dort beschriebenen pflanzlichen oder tierischen Sterin-Verbindungen Gallensäuren als Ausgangsmaterial eingesetzt werden. Im übrigen gelten alle Angaben der genannten europäischen Patentanmeldungen zur Gewinnung der Mikroorganismen-Defektmutanten sowie der nachfolgenden Durchführung des Fermentationsverfahrens mit diesen Mutanten. Vollständigkeitshalber wird im folgenden die Lehre der beiden genannten europäischen Patentanmeldungen in den Grundzügen wiederholt. Zu Einzelheiten wird auf diese Druckschriften verwiesen.

Die Gewinnung der erfindungsgemäß eingesetzten Mikroorganismen-Defektmutanten :

Zur Gewinnung der gewünschten Defektmutanten reihen sich die folgenden vier Verfahrensstufen aneinander :

Stufe 1 : Gezielte Auswahl von Mikroorganismen-Wildstämmen,
Stufe 2 : Mutationsbehandlung,
Stufe 3 : Trennung der gewonnenen Mutantenpopulation aus Stufe 2 bzw. Anreicherung der gewünschten Mutanten, aus der insgesamt gewonnenen Mutantenpopulation,
Stufe 4 : Züchtung der in Stufe 3 abgetrennten bzw. angereicherten Mutantenstämme und Auswahl der Defektmutantenstämme mit optimaler Produktion des gewünschten Verfahrensproduktes.

Diese vier Verfahrensstufen können in einmaligem Durchgang zur Gewinnung der gewünschten Defektmutanten durchlaufen werden. Es kann aber auch zweckmäßig sein, die Verfahrensstufen 2 und 3 ein- oder mehrfach derart zu wiederholen, daß die in Stufe 3 abgetrennte — bzw. angereicherte — gewünschte Mutantenpopulation erneut einer Mutationsbehandlung gemäß Stufe 2 unterworfen und dann wiederum in Stufe 3 aufgetrennt wird.

Im folgenden wird auf die Einzelheiten der aufgezählten Stufen 1 bis 4 eingegangen.

Stufe 1 : Isolierung der geeigneten Wildstämme

Die auszuwählenden Mikroorganismen-Wildstämme sollen befähigt sein, unter aeroben Bedingungen auf 17-C-Alkyl- bzw. -Alkenyl-Sterinverbindungen von der Art des Cholesterins, Sitosterins, Stigmasterins und/oder Ergosterins zu wachsen. Die für die Erfindung geeigneten Wildstämme besitzen dabei vorzugsweise wenigsten etwa die gleiche Abbaugeschwindigkeit für die Seitenkette wie für den Ringanteil der Steroidverbindung. Bevorzugt weisen die Wildstämme jedoch eine erhöhte Seitenkettenabbaugeschwindigkeit im Vergleich mit dem Ringabbau auf.

Die Isolierung der Wildstämme kann dabei in an sich bekannter Weise aus Erdproben erfolgen. Wegen der weiten Verbreitung pflanzlicher und tierischer Sterinverbindungen der angegebenen Art finden sich geeignete Wildstämme praktisch regelmäßig in Erdproben beliebigen Ursprungs. Ihre Züchtung erfolgt unter aeroben Bedingungen in Gegenwart eines wäßrigen Nährmediums in Gegenwart der Sterinverbindung als einziger Kohlenstoffquelle.

Der Auswahl der richtigen Wildstämme kommt eine besondere Bedeutung zu. Dabei ist diese Auswahl auf zweierlei Weise möglich und kann entweder gemäß der Lehre der europäischen Patentanmeldung 004913 oder gemäß der Lehre der europäischen Patentanmeldung 0015308 erfolgen.

Gemäß der Lehre der zuerst genannten Druckschrift werden solche Wildstämme ausgewählt und gezüchtet, die beim Wachstum auf Sterinverbindungen mit gesättigten oder ungesättigten Alkylresten in 17-C-Stellung, und zwar vorzugsweise Resten mit 8 bis 10 C-Atomen, eine selektive Abbauleistung — gemessen unter den im folgenden beschriebenen Standardbedingungen — gemäß der allgemeinen Formel

$$I = a \cdot 10^b$$

ergeben. Hierin ist a der Wachstumsfaktor und b der Selektivitätsfaktor des Wildstammwachstums wie im folgenden definiert. Der Selektivitätsindex I, der für die Auswahl der geeigneten und der bevorzugten Wildstämme charakteristisch ist, ergibt sich somit als Produkt gemäß der angegebenen Formel aus dem Wachstumsfaktor a und dem Selektivitätsfaktor b. Der Zahlenwert von I für die bevorzugten Mikroorganismen-Wildstämme beträgt wenigstens 10. Höhere Zahlenwerte für I sind erwünscht und können beispielsweise bei wenigstens 100 liegen. Bei der Selektion von Wildstämmen aus einer größeren isolierten Wildstammpopulation kann es wünschenswert sein, die Stämme mit den höchsten Zahlenwerten für I den weiteren Stufen des erfindungsgemäßen Verfahrens zu unterwerfen. So kann es besonders zweckmäßig sein, solche Wildstämme auszusuchen, für die der Zahlenwert I wenigstens $10^5$ beträgt.

Die Bestimmung des Wachstumsfaktors a und des Selektivitätsfaktors b des Wildstammwachstums erfolgt dabei unter den folgenden Standardbedingungen (aerobe Verfahrensführung) :

Wachstumsfaktor a :

Der jeweilige Mikroorganismenstamm wird in 500 ml Erlenmeyerkolben (100 ml Nährlösung der im folgenden beschriebenen Zusammensetzung) bei 30 °C auf der Schüttelmaschine (Schüttelfrequenz : 150 UpM) inkubiert. Das Nährmedium hat die folgende Zusammensetzung (pro Liter) :

| | | |
|---|---|---|
| 1,0 | g | $KH_2PO_4$ |
| 0,5 | g | $MgSO_4 \cdot 7\ H_2O$ |
| 0,1 | g | NaCl |
| 0,1 | g | $CaCl_2 \cdot 2\ H_2O$ |
| 5 | g | $(NH_4)_2SO_4$ |
| 1,0 | g | Tween 80 (Polyoxyethylensorbitanmonooleat) |
| 2,0 | g | der 17-C-Seitenkettensterinverbindung, z. B. Cholesterin oder Sitosterin |
| 0,5 | g | Hefeextrakt |
| 0,01 | g | l-Histidin |
| 0,02 | g | dl-Methionin |
| 0,02 | g | dl-Tryptophan |
| 2 | g | Biotin |
| 400 | g | Calciumpantothenat |
| 2 | μg | Folsäure |
| 2 000 | μg | Inosit |
| 400 | μg | Niacin |
| 200 | μg | p-Aminobenzoesäure |
| 400 | μg | Pyridoxinhydrochlorid |
| 200 | μg | Riboflavin |
| 400 | μg | Thiaminhydrochlorid |

Spurenelementlösung
pH-Wert des Nährmediums 6,8.

Zur Messung des Wachstums (Zelldichte) werden in zeitlichen Abständen den Schüttelkulturen aliquote Mengen Zellsuspension entnommen und die jeweilige Extinktion (E') im Zeiss-Photometer (Typ PL 4) bei einer Wellenlänge von nm (Schichtdicke d - 1 cm) gegen destilliertes Wasser gemessen. Die Ausgangsextinktion $E_0$ beträgt vor Beginn des Wachstums — d. h. zum Zeitpunkt $t_0$ — etwa 0,775. Die Suspensionen werden jeweils soweit verdünnt, daß im Bereich $E' = 0,7$ gemessen werden kann.

Der Wachstumsfaktor a ergibt sich als die maximale optische Dichte der Zellsuspension am Ende der logarithmischen Wachstumsphase (Zeitpunkt $t_1$, Extinktion $E_1$), die unter den gegebenen Kulturbedingungen nach spätestens 5 Tagen erreicht wird, d. h. $a = \Delta E = E_1 - E_0$.

Selektivitätsfaktor b :

Die zu prüfenden Stämme werden in Nährbouillon I (Merck) plus 0,1 % Tween 80 (Polyoxyethylensorbitanmonooleat) plus 0,1 % Sterinverbindung, z. B. Cholesterin, 48 Stunden bei 30 °C angezüchtet, die Zellen anschließend mit Puffer gewaschen und in Phosphat-Puffer (pH 7,5) resuspendiert. Der Abbau der Sterinverbindung wird in Parallelansätzen mit radioaktiv markierten Sterinverbindungen, z. B. 4 bis 14 C- und 26 bis 14 C-Cholesterin oder entsprechend radioaktiv markierten Sitosterinverbindungen in Warburg-Gefäßen gemessen.

Zu 2,4 ml Zellsuspension im Hauptgefäß werden 0,2 ml Sterinlösung (z. B. Cholesterinlösung) gegeben (5 $\mu$ Mol mit ca. 0,1 $\mu$Ci markiertem Substrat in 0,1 %iger Tween 80-Lösung), der Ansatz 6 Stunden bei 30 °C inkubiert und die Reaktion anschließend durch Zugabe von 0,2 ml 4n $H_2SO_4$ gestoppt.

Das bei der Substratspaltung entstehende aktive $CO_2$ wird in Ethanolamin (0,2 ml) aufgefangen und im Scintillationscounter gemessen (0,1 ml Proben in jeweils 15 ml Scintillatarflüssigkeit « Unisolv I », Fa. Zinsser).

Der Selektivitätsfaktor b ist das dimensionslose Verhältnis der so gemessenen Radioaktivität

$$b = (^{14}CO_2 \text{ aus Seitenkette von } (26\text{-}^{14}C\text{-})\text{-Sterinverbindung})/(^{14}CO_2 \text{ aus Ringsystem von } (4\text{-}^{14}C)\text{-Sterinverbindung})$$

In dieser Gleichung bedeutet der Begriff « Sterinverbindung » die jeweils eingesetzte radioaktiv markierte Sterinverbindung, also beispielsweise entsprechende Cholesterin- oder ß-Sitosterin-Verbindungen.

Die Technik der Bestimmung des mikrobiologischen Abbaus von Sterinverbindungen durch radioaktive Markierung entweder eines Ringkohlenstoffatoms oder eines Seitenkettenkohlenstoffatoms ist im übrigen eine im Stand der Technik bekannte Methodik, auf deren Einzelheiten hier Bezug genommen wird. Verwiesen wird beispielsweise auf die folgenden Veröffentlichungen : Steroids, 677 - 688 (1964), G. E. Peterson und J. R. Davis « Cholesterol Utilization by Streptomyces spp. » sowie J.B.C. 206, 511-523 (1954) Thressa C. Stadtman et al., « Studies on the Microbiological Degradatuon of cholesterol ».

Für die Auswahl der Wildstämme in der hier beschriebenen Ausführungsform der Stufe 1 des erfindungsgemäßen Verfahrens ist es weiterhin bevorzugt, daß der Selektivitätsfaktor b der Wildstämme wenigstens 1, vorzugsweise wenigstens 2, beträgt. Der Wachstumsfaktor a der Wildstämme sollte wenigstens 0,1, vorzugsweise wenigstens 0,2 betragen und beträgt insbesondere ebenfalls wenigstens 1.

Bei der Auswahl der für die weiteren Stufen des erfindungsgemäßen Verfahrens am besten geeigneten Wildstämme kann es zweckmäßig sein, die Faktoren a und b gegeneinander abzuwägen. So kann es im Rahmen der Erfindung zweckmäßig sein, Wildstämmen mit einem besonders hohen Selektivitätsfaktor b den Vorzug zu geben und bei der Wahl zwischen verschiedenen isolierten Wildstämmen zunächst nach diesem Selektivitätsfaktor b bei möglichst hohen Zahlenwerten für b auszuwählen. Umgekehrt kann natürlich auch ein besonders gutes Wachstum — ausgedrückt durch einen besonders hohen Zahlenwert für den Wachstumsfaktor a — Anlaß sein, für die anschließende Mutation und darauffolgende Selektion der Mutantenpopulation einem solchen Wildstamm den Vorzug zu geben.

Eine Variation und für die Praxis einfachere Arbeitsweise zur Selektion geeigneter Wildstämme gibt die Lehre der europäischen Patentanmeldung 0015308. Gemäß ihrer Anweisung werden solche Mikroorganismen-Wildstämme für die anschließende Mutation, Trennung und schließlich Züchtung der Mutanten-Population ausgewählt, die beim Wachstum auf Sterin-Verbindungen der genannten Art in Gegenwart von Inhibitoren für den enzymatischen Ringabbau der Sterinverbindungen wenigstens anteilsweise 17-C-Steroid-$\alpha$-propionsäureverbindungen liefern.

Inhibitoren für den enzymatischen Ringabbau bei Züchtung von Mikroorganismen auf Sterinverbindungen sind im Stand der Technik ausführlich geschildert, vergleiche hierzu beispielsweise Ch.K.A. Martin « Microbial Cleavage of Sterol Side Chains », Adv. Appl. Microbiol. 22, 29-58 (1977), insbesondere das Unterkapitel IV, B, Seiten 44-50 a.a.O. sowie Nagasawa et al. : Agr. Biol. Chem. 34, 838-844 (1970). Bei der Suche nach geeigneten Mikroorganismen-Wildstämmen können mit überraschend hoher Trefferquote solche Stämme isoliert werden, die in Gegenwart solcher Inhibitoren für den enzymatischen Ringabbau 3-Oxo-pregna-4-en-20-carbonsäure und/oder 3-Oxo-pregna-1,4-dien-20-carbonsäure ($\Delta$-1,4-BNC) liefern.

Als besonders geeignet haben sich in dieser Ausführungsform der Wildstamm-Selektion solche Wildstämme für die anschließende Mutation und Selektion erwiesen, die beim Wachstum auf Sterinverbindungen mit gesättigten oder ungesättigten Alkylresten in 17-C-Stellung mit vorzugsweise 8 bis 10 C-Atomen unter Standardbedingungen eine Ausbeute an 17-C-Steroid-α-propionsäureverbindungen von wenigstens 5 Gewichtsprozent, vorzugsweise von wenigstens 10 Gewichtsprozent — jeweils bezogen auf die im Standardversuch eingesetzte(n) Sterinverbindung(en) — liefern. Die Bedingungen dieses Standardversuchs werden dabei im folgenden im Zusammenhang mit der Bestimmung des Selektivitätsfaktors p beschrieben.

## Ausbeute bzw. Selektivitätsfaktor p des Wildstammes

Der jeweilige Mikroorganismenstamm wird in 500 ml Erlenmeyerkolben (100 ml Nährlösung der im folgenden beschriebenen Zusammensetzung) bei 30 °C auf der Schüttelmaschine (Schüttelfrequenz : 150 U/min) inkubiert. Hier und in allen weiteren Schritten der Mikroorganismen-Züchtung wird unter aeroben Bedingungen gearbeitet.

Das Nährmedium hat die folgende Zusammensetzung (jeweils in Gewichtsprozent) :

0,20 % $K_2HPO_4$
0,05 % $NaH_2PO_4$
0,80 % Pepton
0,90 % Hefeextrakt
0,30 % Glucose
pH 7,2.

Nach 48 Stunden Kulturdauer bei 30 °C erfolgt die Zugabe von 0,1 % Tween 80 (Polyoxyethylensorbitanmonooleat) und 0,1 % 17-C-Seitenketten-Sterinverbindungen, z. B. Cholesterin oder Sitosterin. Nach weiteren 4 Stunden erfolgt die Zugabe des Inhibitors in einer Konzentration von $10^{-3}$ Mol/1 (M).

Zum Zeitpunkt der Inhibitorzugabe wird der pH-Wert des Mediums auf 8,0 justiert. Nach weiterer Inkubation von 62 Stunden wird die Kultur geerntet und der Gehalt an 3-Oxo-pregna-4-en-20-carbonsäure und/oder 3-Oxo-pregna-1,4-dien-20-carbonsäure (Δ-1,4-BNC) nach üblichen Methoden in Gewichtsprozent, bezogen auf eingesetzte Sterinverbindung, bestimmt.

Der Selektivitätsfaktor p errechnet sich dabei aus der prozentualen BNC-Ausbeute gemäß

$$p = (\% \text{ BNC-Ausbeute})/10$$

Innerhalb der Gruppe der in Gegenwart von Inhibitoren BNC bildenden Mikroorganismenstämme werden für die Erfindung in der Regel die Stämme um so geeigneter, je höher die BNC-Ausbeute und damit je größer der Selektivitätsfaktor p im Einzelfall ist. Als unterer Grenzwert für geeignete Wildstämme ist im allgemeinen ein Selektivitätsfaktor p von wenigstens 0,5 anzusehen, wobei jedoch Stämmen mit Selektivitätsfaktoren p von wenigstens 1 oder wenigstens 1,5 der Vorzug gegeben wird. Als besonders geeignet haben sich Stämme mit Selektivitätsfaktoren p von 2 oder mehr erwiesen, wobei nach den besten bisher bekannten Ergebnissen Selektivitätsfaktoren in der Größenordnung von 4 ermittelt worden sind.

Als Inhibitoren zur Hemmung des enzymatischen Abbaus des Sterinringsystems eignen sich aus der hierfür bekannten Gruppe von Verbindungen, beispielsweise α, α′-Dipyridyl, o-Phenanthrolin, 8-Oxochinolin und ganz allgemein Mittel, die unter Chelatbildung Komplexsalze mit Schwermetallen bilden, wie 1-Nitroso-2-naphthol, Salicylaldoxim, Nitrosophenylhydroxylamin, 1-Nitroso-2-naphthol-3,6-disulfonsäure, Tetrahydroxyanthrachinon, und dergleichen. Bevorzugte Inhibitoren für die Durchführung des Standardtestes zur Bestimmung des Selektivitätsfaktors p sind α, α′-Dipyridyl und o-Phenanthrolin, wobei es für die Ausbeutebestimmung nach dem Standardtest ausreichend ist, wenn die geforderte Mindestausbeute mit einem dieser beiden Inhibitoren unter sonst gleichen Züchtungsbedingungen erzielt wird.

Unabhängig von der Bestimmung des Selektivitätsfaktors p erfolgt die bevorzugte Auswahl geeigneter Wildstämme im Rahmen der Erfindung unter zusätzlicher getrennter Bestimmung der Wachstumsgeschwindigkeit der jeweiligen Wildstämme. Hier gelten die vorher gemachten Angaben zur Ermittlung des entpsrechenden Wachstumsfaktors a.

Gemäß dieser Ausführungsform sind für die nachfolgende Mutation und Selektion besonders solche Mikroorganismen-Wildstämme geeignet, die unter Berücksichtigung der zuvor geschilderten Faktoren a und p eine selektive Abbauleistung gemäß der allgemeinen Formel

$$I = a \cdot 10^p$$

(I = Selektivitätsindex) mit einem Zahlenwert von wenigstens 1, vorzugsweise von wenigsten 2 und insbesondere von wenigstens 20 liefern. Auch noch beträchtlich höhere Zahlenwerte können in Betracht kommen, z. B. Werte von $I \geqq 1.010^3$ oder gar $\geqq 5 \cdot 10^3$. Im Rahmen der Erfindung werden dabei Wildstämme mit Wachstumsfaktoren a von wenigstens 0,2, vorzugsweise von wenigstens 1, insbesondere von 4 oder mehr, besonders bevorzugt.

Auch hier kann es zweckmäßig sein, die Faktoren a und p gegeneinander abzuwägen. So kann es im Rahmen der Erfindung zweckmäßig sein, Wildstämmen mit einem besonders hohen Selektivitätsfaktor p den Vorzug zu geben und bei der Wahl zwischen verschiedenen isolierten Wildstämmen zunächst nach diesem Selektivitätsfaktor p bei möglichst hohen Zahlenwerten für p auszuwählen. Umgekehrt kann natürlich auch ein besonders gutes Wachstum — ausgedrückt durch einen besonders hohen Zahlenwert für den Wachstumsfaktor a — Anlaß sein, für die anschließende Mutation und darauffolgende Selektion der Mutantenpopulation einem solchen Wildstamm den Vorzug zu geben.

In Gegenwart von Inhibitoren 3-Oxo-pregna-4-en-20-carbonsäure und/oder 3-Oxo-pregna-1,4-dien-20-carbonsäure ($\Delta$-1,4-BNC)

liefernde Wildstämme finden sich in großer Breite der Mikroorganismen-Klassifikation. Geeignet sind beispielsweise solche von Achromobacter, Arthrobacter, Bacillus, Brevibacterium, Corynebacterium, Flavobacterium, Microbacterium, Mycobacterium, Nocardia, Protaminobacterium, Serratia oder Streptomyces. Diese Klassifikationsangaben haben auch Gültigkeit für die Wildstämme, deren Selektion gemäß der Lehre der europäischen Patentanmeldung 004913 erfolgt.

Nach der Auswahl des geeigneten Wildstammes folgt das weitere Mutations- und Selektionsverfahren. Im einzelnen gilt hierfür :

Mutationsbehandlung (Stufe 2) :

Die Mutation des ausgewählten Wildstammes erfolgt in an sich bekannter Weise. Sie kann beispielsweise durch energiereiche Bestrahlung etwa mit UV oder Röntgenstrahlung erfolgen. Geeignet ist insbesondere aber auch die Behandlung der Zellen mit mutagenen Agentien. Geeignete mutagene Agentien sind beispielsweise Nitrosoguanidinverbindungen, wie 1-Methyl-3-nitro-1-nitrosoguanidin oder Ethylmethansulfonat. Im einzelnen kann hier auf die allgemeinen Angaben des Standes der Technik verwiesen werden, siehe hierzu beispielsweise US-PS 4 029 549, Spalte 2, Zeilen 57 ff mit den dort enthaltenen Verweisen.

Grundsätzlich gilt auch für die Erfindung, daß das Ergebnis der Wildstamm-Mutation insoweit unbestimmt ist, als die Eigenschaften der erhaltenen Defektmutanten im einzelnen nicht vorausbestimmbar sind. Trotzdem lassen sich Prinzipien für eine optimale Mutationsauslösung festlegen, da die Gesetze der Statistik auf eine Mikroorganismenpopulation anwendbar sind. Aus der Literatur sind Verfahren bekannt, die optimalen Bedingungen für die Induktion von Mutationen zu bestimmen (s.a. E.A. Adelberg, M. Mandel, GCC Chen (1965) « Optimal Conditions for Mutagenisis by N-methyl-N'-nitro-N-nitrosoguanidine in Escherichia coli », Biochem. Biophys. Res. Commun. *18*, 788-795).

Im allgemeinen wird zur Erhöhungder Häufigkeit von Mutationen in Mikroorganismenpopulationen die Konzentration und die Einwirkzeit der mutagenen Agentien so gewählt werden, daß bereits ein Teil der Mikroorganismenpopulation letal geschädigt ist. Dabei steigt in dem überlebenden Teil der Population die Häufigkeit der verschiedensten Mutationen mehr oder weniger stark an.

Im Rahmen des erfindungsgemäßen Verfahrens werden die Bedingungen von Konzentration und Einwirkzeit des mutagenen Agens so gewählt, daß die Ausgangspopulation durch die Behandlung zu 10-99,999 % inaktiviert wird. Vorzugsweise wird eine Abtötungsrate von 90-99,99 % gewählt.

An die Verfahrensstufe 2 der Mutation schließen sich die Verfahrensstufen 3 und 4 an.

Zu Stufe 3

Zur nachfolgenden Auslese der erfindungsgemäß bestimmt gewünschten Mutanten aus der großen Population der Mikro-organismen nach der Mutationsbehandlung werden erfindungsgemäß Kulturbedingungen gewählt, unter denen die abgeänderten spezifischen Eigenschaften der entstandenen Mutantenstämme zum Selektionsvorteil werden. Die Anreicherung der erwünschten Defektmutanten erfolgt erfindungsgemäß häufig unter Bedingungen, unter denen die spezielle Mutante nicht oder praktisch nicht wächst, während unerwünschte Begleitorganismen wachsen und durch ihr Wachstum oder bei ihrem Wachstum abgetötet werden. Auf diese Weise gelingt es, diejenigen Defektmutantenstämme zu isolieren, deren ringabbauende Enzyme blockiert sind, jedoch nach wie vor zum Abbau der 17-C-Seitenkette an der Sterinverbindung befähigt sind, wobei durch weitere Maßnahmen insbesondere dieser Stufe 3 sichergestellt wird, daß gerade solche Defektmutanten isoliert werden können, die beim Seitenkettenabbau bevorzugt den $\alpha$-Propionsäurerest in 17-C-Stellung ausbilden.

Hierzu wird in der Verfahrensstufe 3 die Mutantenpopulation auf einem « Trennmedium » gezüchtet, das letztlich als Anreicherungsmedium für die gewünschten Mutantenstämme dient. Als Mutanten-Trennmedium kann ein wäßriges Nährmedium eingesetzt werden, das als Kohlenstoffquelle 3-Oxo-pregna-4-en-20-carbonsäure und/oder 3-Oxo-pregna-4-en-20-carbonsäure enthält.

Im übrigen wird hier mit konventionellen Nährlösungen unter aeroben Bedingungen gearbeitet.

Diejenigen mutierten Mikroorganismen, die aufgrund der Mutationsbehandlung ihre Fähigkeit verloren haben, auf den jetzt vorliegenden Kohlenstoffquellen zu wachsen, können sich beim Bebrüten des Mutantentrennmediums nicht vermehren. Sie wachsen also nicht oder nicht wesentlich. Ein anderer Teil der Mutantenpopulation, der entweder bei der Mutationsbehandlung der Stufe 2 nicht hinreichend geschädigt worden ist oder andere Defekte erlitten hat, ist befähigt, auf der Kohlenstoffquelle des Mutantentrennmediums zu wachsen. Bei der Bebrütung tritt hier also eine Vermehrung ein.

Die Erfindung macht sich dieses unterschiedliche Verhalten derart zunutze, daß die Bedingungen im Mutantentrennmedium so gewählt werden, daß die wachsenden Stämme aufgrund ihres Wachstums oder während ihres Wachstums abgetötet werden, ohne daß dabei die nicht wachsenden Mutantenstämme geschädigt werden.

Möglich ist eine solche Trennung beispielsweise durch Zusatz von Antibiotika, beispielsweise durch Zusatz von Penicillinverbindungen. Die Zugabe von Penicillinverbindungen führt zur Abtötung des Anteils der wachsenden Mikroorganismenstämme, während die nicht wachsenden Stämme ungeschädigt bleiben.

Aus den verbliebenen ungeschädigten Blockmutanten können dann z. B. mittels der an sich bekannten Lederbergschen Stempelmethode die erfindungsgemäß angestrebten Defektmutantenstämme isoliert werden. Bezüglich der hier eingesetzten Verfahrenstechnik und zur Penicillinanreicherungsmethode und zur Lederbergschen Stempelmethode wird biespielsweise verwiesen auf J. Amer. Chem. Soc. 70, 4267, (1948) Davis, B.D. (Penicillin-Anreicherungsmethode), J. Bact. 63, 399 (1952), Lederberg, J., Lederberg, E.M. (Lederbergsche Stempelmethode).

Eine andere geeignete Methode ist die Abtötung der ungeeigneten Mutanten durch Verwendung von Nährmedien, die radioaktive Komponenten enthalten, welche letztlich die wachsenden Zellen insbesondere unter Einbau in die Zellstruktur schädigen. Geeignet ist hier beispielsweise die Abtötung der ungeeigneten Mutanten durch ein Nährmedium, das $^{32}P$ insbesondere in Form des Salzes $NaH_2{}^{32}PO_4$ enthält, zu dieser Inaktivierungstechnik siehe Fuerst, C.R., Stent, G.S. : Inactivation of Bacteria by Decay of Incorporated Radioactive Phosphorus, J. Gen. Physiol. 40, 73-90 (1956).

Zu Stufe 4

Die so isolierten und selektierten Defektmutanten können jetzt auf einem üblichen Nährmedium angezüchtet werden und dann gewünschtenfalls einer weiteren Selektion unterworfen werden. Die Selektion kann hier beispielsweise nach dem angestrebten Ergebnis der Mikroorganismen-Stämme auf dem beabsichtigten Einsatzmaterial, beispielsweise also auf natürlichen oder pflanzlichen Sterinverbindungen erfolgen, wobei hier insbesondere die chemische Natur der Stoffwechselprodukte des Mikroorganismen-Wachstums und die Wachstumsfreudigkeit der Stämme bestimmend sein können. Naturgemäß wird man Defektmutanten mit optimaler Produktivität des letztlich gewünschten Verfahrensprodukts bevorzugen. Diese Stämme können dann im technischen Verfahren eingesetzt werden. Anzüchtung und Selektion können hier einfach oder mehrfach wiederholt werden.

Es kann bevorzugt sein, die besonders erwünschten Defektmutantenstämme durch ihre Transformationsleistung in einem Standardtest zu ermitteln. Bestimmt wird hierbei die Leistungsfähigkeit des Stammes bezüglich der Bildung von 3-Oxo-pregna-4-en-20-carbonsäure (Δ-4-BNC) und/oder 3-Oxo-pregna-1,4-dien-20-carbonsäure (Δ-1,4-BNC). Für die Ausbeutungsbestimmung nach diesem Standardtest gelten dabei die folgenden Bedingungen :

BNC-Ausbeute nach Standardtest

Der zu prüfende Defektmutantenstamm wird im 500 ml Erlenmeyerkolben mit 100 ml Nährlösung folgender Zusammensetzung gezüchtet : 0,8 % Pepton, 0,9 % Hefeextrakt, 0,3 % Glukose, 0,06 % Tween 80 (Polyoxyethylensorbitanmono-oleat), 0,06 % Sterinverbindung (Cholesterin oder Sitosterin), pH 7,2. Die Kultur wird auf der Schüttelmaschine (Schüttelfrequenz 150 UpM) bei 30 °C für 62 Stunden vorgezüchtet. Anschließend werden 0,1 % BRIJ 35 (Polyoxyethylenmonolaurylether) und 0,1 % der zuvor gewählten Sterinverbindung (Cholesterin oder Sitosterin) zugegeben und für weitere 120 Stunden bebrütet. Nach Abbruch der Kulturen werden Proben genommen und diese extrahiert und dünnschichtchromatographisch analysiert. Die prozentuale Ausbeute an 3-Oxo-pregna-4-en-20-carbonsäure und/oder 3-Oxo-pregna-1,4-dien-20-carbonsäure wird bestimmt. Sämtliche Prozentangaben sind Gewichtsprozent. Die Ausbeute bezieht sich auf die im Standardversuch eingesetzte Gewichtsmenge der zu transformierenden Sterinverbindung.

Die im Sinne der Erfindung bevorzugten Defektmutantenstämme liefern unter diesen Standardbedingungen mit Cholesterin oder Sitosterin als Steroidsubstrat eine Mindestausbeute von 15 Gewichtsprozent, bevorzugt Ausbeuten von 30 Gewichtsprozent und mehr. Bei der Verwendung von Cholesterin als Steroidsubstrat kann die bevorzugte Mindestausbeute im Sinne dieses Standardtestes 50 Gewichtsprozent oder mehr ausmachen, wobei besonders wirkungsvolle Stämme BNC-Ausbeuten im Bereich von 70 bis 80 Gewichtsprozent liefern. Bei der Verwendung von Sitosterin als Steroidsubstrat kann die Ausbeute etwas niedriger liegen, aber auch hier Werte im Bereich von 40 bis 50 Gewichtsprozent erreichen.

Das erfindungsgemäße Arbeiten mit den Mikroorganismen-Defektmutanten

Die erfindungsgemäße selektive Umwandlung der als Ausgangsmaterial gewählten Gallensäuren kann nach Schaffung und Auswahl der Defektmutanten in an sich bekannter Weise erfolgen — siehe auch hierzu die vergleichbaren Angaben der europäischen Offenlegungsschriften 004913 und 0015308. So kann also beispielsweise das Einsatzmaterial der Kultur während der Inkubationsperiode zugesetzt werden oder es kann dem Nährmedium vor der Inokulierung der Defektmutanten beigegeben werden. Es kann eine bestimmte Gallensäure oder auch eine Mischung mehrerer Gallensäuren eingesetzt werden. Vorzugsweise werden die selektiv abzubauenden Steroidverbindungen in der Kultur in Mengen von etwa 0,1 bis 100 g/l verwendet. Die optimale Konzentration der zu transformierenden Gallensäuren in der Züchtungsstufe ist im allgemeinen Stamm-abhängig und kann durch einfache Vorversuche jeweils ermittelt werden. Im allgemeinen liegt ihre Konzentration vorzugsweise nicht über 50 g/l und häufig nicht über 30 g/l, jedoch werden Mengen über 1 g/l bevorzugt.

Es kann dabei bevorzugt sein, das dem Seitenkettenabbau zu unterwerfende Substrat nicht auf einmal dem Reaktionsmedium zuzusetzen, sondern diese Zugabe nach und nach im Verlauf der Reaktion vorzunehmen. Vorzugsweise wird das Ausgangssubstrat in dieser Ausführungsform im wesentlichen kontinuierlich im Reaktionsgemisch im Verlauf der Abbaureaktion zugegeben. Auf diese Weise kann häufig die Ausbeute der gewünschten Abbauprodukte erhöht werden.

Die Kultur wird in einem Nährmedium gezüchtet, das als Kohlenstoffquelle entweder die zu transformierenden Gallensäuren oder aber auch noch zusätzliche metabolisierbare Kohlenstoffquellen sowie die üblicherweise von diesen Mikroorganismen benötigten Nähr- und Wuchsstoffe enthält. Besonders günstig für das Wachstum der Mikro-organismen sind z. B. Paraffin, Glycerin, Carbonsäuren, Stärke, Dextrin, Saccharose, Glucose, Fructose und zuckerhaltige Abfallstoffe. Geeignete Stickstoffquellen sind Ammoniumsalze, Nitrate, Pepton, Maisquellwasser, Sojamehl, Schlempe und Fischmehl. Weiterhin können Fermentationsbeschleuniger, wie Hefeextrakt und Vitamine zugesetzt werden. Das Nährmedium enthält darüber hinaus zweckmäßigerweise anorganische Salze, wie Natrium-, Kalium- oder Ammoniumphosphate sowie Calcium-, Magnesium-, Mangan- oder Eisensalze.

Die Emulgierung der Gallensäuren in dem Nährmedium erfolgt vorzugsweise mittels bekannter Emulgatoren, z. B. mittels Fettsäure-Sorbitanester oder deren Ethylenoxidaddukten, Polyoxyethylenmonolaurylether oder Fettsäureamidoalkylbetain.

Das verwendete Kulturmedium wird vor Beginn der Bakterienanzucht zweckmäßigerweise durch Erhitzen sterilisiert. Nach dem Abkühlen und Beimpfen des Kulturmediums mit einer geeigneten Vorkultur des transformierenden Bakterienstammes wird zwischen 25 bis 55 °C inkubiert, vorzugsweise bei 27 bis 30 °C. Der pH-Wert der Nährlösung liegt zwischen pH 4 bis 8,5 vorzugsweise bei 7,0 bis 8,0. Die Kultur wird durch Schütteln, Rühren oder Gaseinleiten mit Sauerstoff versorgt. Der Abbau der Gallensäuren fordert je nach Substratkonzentration und den anderen Fermentationsbedingungen in der Regel 24 bis 160 Stunden. Die erfindungsgemäße Auswahl der Defektmutanten ermöglicht das Arbeiten in Abwesenheit von Inhibitoren für den Steroid-Ringabbau. Hierdurch können eine beträchtliche Steigerung der Ausbeute und/oder Beschleunigung der Reaktion erreicht werden.

Das auf diese Weise hergestellte Verfahrensprodukt, das sich üblicherweise in der Fermentationsbrühe anreichert, kann dann in an sich bekannter Weise aus dem Reaktionsgemisch gewonnen werden. Es kann beispielsweise durch Extraktion mit organischen Lösungsmitteln, wie Methylisobutylketon, Essigsäureester, n-Hexanol, n-Octanol, Chloroform oder n-Hexan, aus dem Kulturmedium vor oder nach Abtrennen der Zellen isoliert werden.

Beispielsweise gelingt eine Isolierung der gebildeten BNC-Verbindungen, indem man einen Liter einer Fermentationslösung, die 1 g Reaktionsprodukt enthält, mit etwa gleichen Volumina organischer Lösungsmittel, wie mit Methylisobutylketon, Essigsäureester, n-Hexanol, n-Octanol, Chloroform oder n-Hexan im Perforator, Turborührer oder Scheidetrichter extrahiert. in den beiden letztgenannten Fällen muß zur Abtrennung der BNC-haltigen organischen Phase aus der Emulsion ein Zentrifugationsschritt angeschlossen werden.

Die Lösungsmittelextraktion ist insbesondere im sauren pH-Bereich möglich. Dazu wird beispielsweise mit 50 %iger $H_2SO_4$ etwa auf pH 2 eingestellt und mit Methylisobutylketon, Essigsäureester, n-Hexanol, n-Octanol, Chloroform oder n-Hexan in der zuvor angegebenen Weise extrahiert. Nach Eindampfen der organischen Phase wird auch hier ein Rückstand erhalten, der wiederum nach Umkristallisation zum Reinprodukt aufgearbeitet werden kann. Die Extraktion kann auch im neutralen Bereich durchgeführt werden.

Alternativ ist auch eine Reinigung durch Anionenaustausch möglich. Hierzu wird die Fermentationslösung zweckmäßigerweise zunächst aufkonzentriert und dann auf einen alkalischen Wert, beispielsweise pH 12, eingestellt. Nach Zusatz einer beschränkten Menge Methanol und Verrühren im Turborührer wird die Zellmasse mittels einer Durchlaufzentrifuge abgetrennt. Der wäßrig-methanolische Überstand wird über eine Ionenaustauschersäule gepumpt, die mit einem Anionenaustauscherharz, z. B. in Acetatform, gefüllt ist. Das gebildete Produkt wird dabei vollständig im Ionenaustauscher gebunden. Durch Elution mit 10 % iger Essigsäure in Methanol gelangt man zu einem Rohprodukt, das z. B. durch Umkristallisation gereinigt werden kann.

Nach einer bevorzugten Ausführungsform gelingt die Isolierung des Verfahrensproduktes aus der Fermenterflüssigkeit ganz einfach durch Ausfällung im sauren Bereich und Abfiltrieren. Hierzu wird die alkalisch eingestellte Fermenterflüssigkeit zunächst filtriert, um das Zellmaterial und andere feste Bestandteile zu entfernen. Anschließend wird angesäuert, dabei fällt das Verfahrensprodukt in fester filtrierbarer Form aus und kann z. B. durch einfaches Abnutschen gewonnen werden.

Zum Ansäuern ist jede Mineralsäure verwendbar, es können aber auch organische Säuren. z. B.

Essigsäure, und sogar gasförmiges $CO_2$ eingesetzt werden. Bei pH 5 ist die praktisch vollständige Ausfällung erreicht. Zur besseren Filtrierbarkeit kann es günstig sein, die Suspension kurz zu erhitzen. Der isolierte Feststoff kann bis zu 90 % des gewünschten Produktes enthalten. Die reinen Verbindungen lassen sich durch Umkristallisation daraus gewinnen.

Im Rahmen der Erfindung besonders geeignete Mikroorganismen-Wildstämme und Mutantenstämme, ihre Hinterlegungsstelle und ihr Hinterlegungsdatum sind nachfolgend genannt :

CBS = Centraalbureau voor Schimmelcultures, Baarn, Niederlande
DSM = Deutsche Sammlung von Mikroorganismen, Grisebachstraße 8, D-3400 Göttingen

| Wildstämme | | Mutantenstämme | |
|---|---|---|---|
| CBS 660.77 | 27.12.1977 | | |
| DMS 1418 | 08.01.1979 | | |
| DSM 1419 | 08.01.1979 | CBS 437.77 | 31.08.1977 |
| DSM 1421 | 08.01.1979 | DSM 1435 | 08.01.1979 |
| DSM 1423 | 08.01.1979 | DSM 1437 | 08.01.1979 |
| DSM 1424 | 08.01.1979 | DSM 1439 | 08.01.1979 |
| DSM 1425 | 08.01.1979 | DSM 1442 | 08.01.1979 |
| DSM 1426 | 08.01.1979 | DSM 1443 | 08.01.1979 |
| DSM 1427 | 08.01.1979 | DSM 1444 | 08.01.1979 |
| DSM 1428 | 08.01.1979 | DSM 1445 | 08.01.1979 |
| DSM 1429 | 08.01.1979 | | |
| DSM 1430 | 08.01.1979 | | |
| DSM 1431 | 08.01.1979 | | |
| DSM 1432 | 08.01.1979 | | |

Beispiel

Der Stamm DSM 1444 wird im 7 l-Fermenter (5 l Füllvolumen) im folgenden Nährmedium bei 30 °C aerob angezüchtet :

0,50 % Glucose
0,30 % Hefeextrakt (KAV)
0,80 % Maisquellwasser
1,80 % Maiskleber
0,15 % $K_2HPO_4$
0,09 % $(NH_4)_2 HPO_4$

Der pH des Nährmediums beträgt 7,0. Die Prozentangaben beziehen sich durchweg auf Gewichtsprozent.

Nach 24-stündiger Inkubation erfolgt die Zugabe von 0,05 Gewichtsprozent eines Emulgiermittels (DK Ester F 50 zusammen mit 0,1 Gewichtsprozent Desoxycholsäure. Nach weiteren 8 Stunden werden erneut 0,5 Gewichtsprozent des gleichen Emulgiermittels zusammen mit 1,9 Gewichtsprozent Desoxycholsäure und 1,0 Gewichtsprozent Glucose über einen Zeitraum von 48 Stunden zudosiert.

Nach 96 Stunden Fermentationszeit wird der Ansatz abgebrochen, die Zellmasse abfiltriert, die zellfreie Fermenterbrühe im Dünnschichtverdampfer im Verhältnis 3 : 1 aufkonzentriert und das Konzentrat mit $H_2SO_4$ auf pH 2,0 eingestellt. Anschließend wird mit Methylenchlorid im Volumverhältnis 1 : 1 extrahiert. Nach Eindampfen des Methylenchlorids wird ein Rohprodukt erhalten, das als Hauptkomponente etwa 45 g 12-Oxo-BNC und 25 g 12-α-hydroxy-BNC enthält.

**Patentansprüche**

1. Verfahren zum mikrobiologischen Abbau von Gallensäuren durch aerobes Züchten von Mikroorganismen in einem wäßrigen Medium unter Verwendung eines Gallensäuren enthaltenden Einsatzmaterials als Kohlenstoffquelle für das Mikroorganismen-Wachstum, dadurch gekennzeichnet, daß man zum selektiven Abbau der Steroid-C17-carboxyalkylseitenkette zum α-Propionsäurerest und zur gleichzeitigen Umwandlung des Steroid-A-Ringes in die 3-Oxo-4-en- und/oder 3-Oxo-1,4-dien-Struktur mit Mikroorganismen-Defektmutanten arbeitet, die zum Wachstum auf pflanzlichen oder tierischen Sterinverbindungen mit einer C17-Alkyl- bzw. -Alkenylseitenkette als alleiniger Kohlenstoffquelle befähigt sind und aus diesen auch in Abwesenheit von den Steroidringabbau- und/oder das Wachstum hemmenden Inhibitoren 3-Oxo-pregna-4-en-20-carbonsäure und/oder 3-Oxo-pregna-1,4-dien-20-carbonsäure bilden.

2. Verfahren nach Anspruch 1, dadurch gekennzeichnet, daß man mit Defektmutanten arbeitet, welche durch Mutation und Selektion aus solchen Wildstämmen gewonnen worden sind, die beim aeroben Wachstum unter Standardbedingungen auf Sterin-Verbindungen mit gesättigten oder ungesättigten Alkylresten mit 8 bis 10 C-Atomen in C-17 in Gegewart von Inhibitoren für den enzymatischen Ringabbau eine selektive Abbauleistung I von wenigstens 1, vorzugsweise von wenigstens 2 und insbesondere von wenigstens 20 ergeben, wobei die selektive Abbauleistung bestimmt ist als

$$I = a \cdot 10^P$$

worin a der Wachstumsfaktor und p der Selektivitätsfaktor des Wildstammwachstums sind.

3. Verfahren nach Ansprüchen 1 und 2, dadurch gekennzeichnet, daß man mit Defektmutanten arbeitet, die bei Züchtung auf Cholesterin unter Standardbedingungen eine Mindestausbeute an 3-Oxo-pregna-4-en-20-carbonsäure und/oder 3-Oxo-pregna-1,4-dien-20-carbonsäure von 50 % — bezogen auf das Gewicht des eingesetzten Cholesterins — liefern.

4. Verfahren nach Ansprüchen 1 bis 3, dadurch gekennzeichnet, daß man mit Defektmutanten von Achromobacter, Arthrobacter, Bacillus, Brevibacterium, Corynebacterium, Flavobacterium, Microbacterium, Mycobacterium, Nocardia, Protaminobacterium, Serratia oder Streptomyces arbeitet.

5. Verfahren nach Ansprüchen 1 bis 4, dadurch gekennzeichnet, daß man mit einem der folgenden Defektmutantestämme arbeitet : CBS 437.77, DSM 1435, DSM 1437, DSM 1439, DSM 1442, DSM 1443, DSM 1444 oder DSM 1445.

## Claims

1. A process for the micorbiological degradation of bile acids by aerobic culture of microorganisms in an aqueous medium using a starting material containing bile acids as carbon source for the growth of the microorganisms, characterized in that, for selective degradation of the steroid-C17-carboxyalkyl side chain to the α-propionic acid residue and for simultaneous conversion of the steroid-A ring into the 3-oxo-4-ene and/or the 3-oxo-1,4-diene structure, defective microorganism mutants are used which are capable of growth on vegetable or animal sterol compounds containing a C17-alkyl or alkenyl side chain as sole carbon source and which form 3-oxopregna-4-ene-20-carboxylic acid and/or 3-oxopregna-1,4-diene-20-carboxylic acid from those compounds, even in the absence of inhibitors inhibiting degradation of the steroid ring and/or growth.

2. A process as claimed in Claim 1, characterized in that the defective mutants used have been obtained by mutation and selection from wild strains of the type which, on aerobic growth under standard conditions on sterol compounds containing saturated or unsaturated alkyl groups containing from 8 to 10 C-atoms in C-17 in the presence of inhibitors for enzymatic ring degradation, have a selective degradation capacity I of at least 1, preferably of at least 2 and more preferably of at least 20, the selective degradation capacity being defined as

$$I = a \cdot 10^P$$

where a is the growth factor and p the selectivity factor of the growth of the wild strain.

3. A process as claimed in Claims 1 and 2, characterized in that defective mutants are used which, in the event of growth on cholesterol under standard conditions, give a minimum yield of 3-oxopregna-4-ene-20-carboxylic acid and/or 3-oxopregna-1,4-diene-20-carboxylic acid of 50 %, based on the weight of the cholesterol used.

4. A process as claimed in Claims 1 to 3, characterized in that defective mutants of Achromobacter, Arthrobacter, Bacillus, Brevibacterium, Corynebacterium, Flavobacterium, Microbacterium, Mycobacterium, Nocardia, Protaminobacterium, Serratia or Streptomyces are used.

5. A process as claimed in Claims 1 to 4, characterized in that one of the following defective mutant strains is used : CBS 437.77, DSM 1435, DSM 1437, DSM 1439, DSM 1442, DSM 1443, DSM 1444 or DSM 1445.

## Revendications

1. Procédé de dégradation microbiologique d'acides biliaires, par culture aérobie de microorganismes dans un milieu aqueux, avec utilisation d'une matière de départ contenant un acide biliaire, comme source de carbone, pour la croissance des microorganismes, procédé caractérisé en ce que, pour réaliser la dégradation sélective de la chaîne carboxy-alcoyle latérale de stéroïde-C17 en reste d'acide α-propionique, et la transformation simultanée du cycle stéroïde-A en structure 3-Oxo-4-ène et/ou 3-Oxo-1,4-diène, on utilise des microorganismes mutants par défaut, dont la croissance nécessite comme source unique de carbone des composés stéroliques d'origine végétale ou animale avec une chaîne latérale alcoylique ou alcénique en C17, à partir desquels ils forment, même en l'absence d'inhibiteurs

11

ralentissant la dégradation du cycle stéroïdique et/ou la croissance, des acides 3-Oxo-pregna-4-ène-20-carboxylique et/ou 3-Oxo-pregna-1,4-diène-20-carboxylique.

2. Procédé selon la revendication 1, caractérisé en ce que l'on utilise des mutants par défaut qui ont été obtenus par mutation et sélection à partir de souches non cultivées qui, au cours de la croissance aérobie dans des conditions standards, donnent sur des composés stéroliques avec des restes alcoyle saturés ou insaturés comprenant 8 à 10 atomes de C en C-17, en présence d'inhibiteurs de la dégradation enzymatique de cycle, une capacité de dégradation sélective I d'au moins 1, préférence d'au moins 2 et en particulier d'au moins 20, la capacité de dégradation sélective étant déterminée comme

$$I = a \cdot 10^P$$

où a est le facteur de croissance et P le facteur de sélectivité de la croissance de la souche non cultivée.

3. Procédé selon les revendications 1 et 2, caractérisé en ce que l'on utilise des mutants par défaut qui, au cours de la culture sur cholestérol dans des conditions standard, produisent un rendement minimum en acides 3-Oxo-pregna-4-ène-20-carboxylique et/ou 3-Oxo-pregna-1,4-diène-20-carboxylique de 50 %, calculé sur le poids de cholestérol mis en œuvre.

4. Procédé selon les revendications 1 à 3, caractérisé en ce que l'on opère avec des mutants par défaut des achromobacter, arthrobacter, bacillus, brevibacterium, corynebacterium, flavobacterium, microbacterium, mycobacterium, nocardia, protaminobacterium, serratia ou streptomyces.

5. Procédé selon les revendications 1 à 4, caractérisé en ce que l'on utilise une des souches suivantes de mutants par défaut : CBS 437.77, DSM 1435, DSM 1437, DSM 1439, DSM 1442, DSM 1443, DSM 1444 ou DSM 1445.